# NEUE EUROPÄISCHE PATENTSCHRIFT

(11) **EP 1 868 520 B2**
(45) Veröffentlichungstag und Bekanntmachung des Hinweises auf die Entscheidung über den Einspruch: **24.07.2019**
(45) Hinweis auf die Patenterteilung: 01.09.2010
(21) Anmeldenummer: 06707558.0
(22) Anmeldetag: 14.03.2006
(51) Int. Cl.: A61B 18/14, A61B 17/32, A61B 17/22

(54) **Endoskopisches Chirurgiegerät**
Endoscopic surgical instrument
Instrument chirgurgical d'endoscopie

(30) Priorität: 11.04.2005 DE 102005016602; 04.05.2005 DE 102005020948; 16.08.2005 DE 102005038694
(43) Veröffentlichungstag der Anmeldung: 26.12.2007
(73) Patentinhaber: Erbe Elektromedizin GmbH, 72072 Tübingen (DE)
(72) Erfinder: FISCHER, Klaus, 72202 Nagold (DE); QUECK, Jochen, 72076 Tübingen (DE); KALTHOFF, Friedrich, 58540 Meinerzhagen (DE)
(74) Vertreter: Bohnenberger, Johannes
(86) Internationale Anmeldenummer: PCT/EP2006/002328
(87) Internationale Veröffentlichungsnummer: WO 2006/108480

(56) Entgegenhaltungen:
- EP-A- 0 280 972
- EP-A2- 0 893 101
- EP-B1- 0 555 549
- WO-A1-96/36381
- DE-A1- 3 642 077
- DE-A1- 4 242 143
- DE-A1- 10 028 413
- DE-A1- 19 607 922
- JP-A- H05 212 045
- US-A- 5 116 313
- US-A- 5 505 729
- US-A- 5 741 271
- US-A- 6 010 512
- US-A- 6 123 665
- US-A1- 2003 216 724
- US-B1- 6 689 126

## Beschreibung

Die Erfindung betrifft ein endoskopisches Chirurgiegerät, insbesondere für die endoskopische Mukosaresektion. JP05-212045 offenbart ein zweilumiges Endoskop, wobei in einem Lumen eine Injektionsnadel und in einem anderen Lumen eine Hochfrequenz-Elektrode angeordnet ist, die unabhängig voneinander aus dem Endoskop herausschiebbar sind.

Die Resektion großflächiger Tumore im Gastrointestinaltrakt, die auf die Mukosa begrenzt sind, sollen möglichst in einer Sitzung und möglichst vollständig ektomiert werden. Dazu wird üblicherweise die Schlingentechnik oder die Kappentechnik eingesetzt, bei der jedoch nur in Abhängigkeit vom Schlingendurchmesser bzw. vom Kappendurchmesser unterschiedlich große kreisrunde Resektate erstellt werden. Großflächige Tumore mit einem Durchmesser von mehr als 8 Zentimetern können daher nur in der sogenannten Piecemeal-Technik abgetragen werden. Es wird jedoch angestrebt; auch großflächige Tumore komplett ektomieren zu können. Dazu werden bereits in der Praxis erste Verfahren erprobt, wobei mit einem flexiblen Nadelmesser die Mukosa Stück für Stück umschnitten und dann komplett entfernt wird. Die Resektion muss dabei in der Submukosa erfolgen. Die Schichtdicken betragen ungefähr 0,5 - 1,5 Millimeter.

Dabei kann es zu Blutungen und Perforationen der Darm- bzw. Magenwand kommen.

Um hier Abhilfe zu schaffen, wird bei der endoskopischen Mukosaresektion vor der Resektion zuerst die Mukosa durch eine flexible Nadel mit Flüssigkeit unterspritzt. Die Nadel muss dabei genau in der Submukosa platziert werden. Durch das Eindringen der Flüssigkeit in die Mukosa wird diese deutlich von der Muscularis Propria abgelöst, wobei ein Flüssigkeitskissen unter der Mukosa entsteht. Dadurch wird ein Sicherheitsabstand zur Muscularis Propria sowie ein Wärmeschutz gewonnen. Mit einem flexiblen Nadelmesser wird dann die Mukosaresektion durchgeführt. Die Resektion gestaltet sich äußerst schwierig, so dass die Operationszeit für derartige Eingriffe derzeit zwischen 2 bis 6 Stunden beträgt

Die Schwierigkeit bei derartigen Eingriffen besteht insbesondere darin, dass die injizierte Flüssigkeit aus der Submukosa während der Operation langsam entweicht, so dass durch die Nadel die Muskolaris Propria thermisch beschädigt werden kann. Dies wiederum kann zu einer Perforation des Darmes führen. Um dies zu vermeiden, muss während des Resektionsvorganges das Instrument mehrmals gewechselt und die Mukosa wieder unterspritzt werden.

Eine Vorrichtung, die zum Unterspritzen durch endoskopisches Injizieren einer Flüssigkeit geeignet ist, wird beispielsweise in der DE 19 607 922 C2 beschrieben.

Es werden ferner alternative Lösungen getestet, bei denen Flüssigkeiten mit unterschiedlicher Viskosität verwendet werden, die weniger schnell aus der Mukosa entweichen. Diese Lösungsansätze bieten aber keine befriedigende Lösung, da das Entweichen der Flüssigkeit aus der Submukosa lediglich verzögert wird.

Ferner sind chirurgische Vorrichtungen bekannt, die eine Resektion bzw. Dissektion von Gewebe durch HF-Strom bewirken. Derartige endoskopische HF-Chirurgiegeräte sind beispielsweise in US 6,123,665 A, US 6,010,512 A und US 5,741,271 A beschrieben.

EP 02 80 972 A1 offenbart ferner eine Flüssigkeitsstrahleinrichtung, die ein eine Düse bildendes Endstück umfasst, wobei das Endstück eine HF-Elektrode umfasst. Die Flüssigkeitsstrahleinrichtung ist also mit der HF-Elektrode integral ausgebildet, so dass das Endstück einerseits starr ist und andererseits einen relativ großen Querschnittsdurchmesser aufweist, wodurch die Handhabung der bekannten Flüssigkeitsstrahleinrichtung erschwert ist.

Der Erfindung liegt die Aufgabe zugrunde, ein Chirurgiegerät anzugeben, mit dessen Hilfe endoskopische Eingriffe, wie beispielsweise die Mukosaresektion vereinfacht wird, wobei gleichzeitig die Schädigung des umliegenden Gewebes, insbesondere der Magen- oder Darmwand, sicher vermieden werden soll.

Erfindungsgemäß wird diese Aufgabe durch ein endoskopisches Chirurgiegerät mit den Merkmalen des Anspruchs 1 gelöst.

Ein wesentlicher Punkt der Erfindung besteht demnach darin, das endoskopische Chirurgiegerät mit einer Einrichtung zu Zufuhr mindestens einer Flüssigkeit, insbesondere zur Injektion und/oder Dissektion, und einer Einrichtung zur Hochfrequenz-Chirurgie zu versehen, wobei die Einrichtung zur Hochfrequenz-Chirurgie mit der Einrichtung zur Zufuhr der mindestens einen Flüssigkeit zur einheitlichen Handhabung kombiniert ist.

Dadurch wird ein Multifunktionsgerät bereitgestellt, das die Vorteile der Wasserstrahlchirurgie umfassend die Injektion der Flüssigkeit und/oder die Dissektion mittels der Flüssigkeit, mit den Vorteilen der Hochfrequenz-Chirurgie verbindet. Das erfindungsgemäße Chirurgiegerät gestattet es, mit dem Wasserstrahl die Mukosa dosierbar zu unterspritzen und diese dadurch von der Muscularis abzuheben. Außerdem kann mit dem Wasserstrahl bei einem entsprechenden Druck die Mukosa durchtrennt werden, so dass mit dem erfindungsgemäßen Chirurgiegerät bereits zwei Funktionen, nämlich die Injektion der Flüssigkeit in die Submukosa sowie die Resektion der Mukosa erfüllt werden.

Um die Submukosa zu trennen, wird die Einrichtung zur Hochfrequenz-Chirurgie eingesetzt.

Es ist auch möglich, mit Hilfe des erfindungsgemäßen Chirurgiegerätes die Resektion der Mukosa anstelle mit Hilfe der Flüssigkeit durch die Einrichtung zur Hochfrequenz-Chirurgie vorzunehmen, so dass die Einrichtung zur Zufuhr der mindestens einen Flüssigkeit lediglich zur Injektion und somit zum Abheben der Mukosa eingesetzt wird.

Der Vorteil des erfindungsgemäßen Chirurgiegerätes besteht somit insbesondere darin, dass mit Hilfe eines einzigen Gerätes die Funktionen Unterspritzen der Mukosa, Trennen der Mukosa und Trennen der Submukosa durchgeführt werden können. Ein Instrumentenwechsel während der Operation ist damit nicht mehr erforderlich. Vielmehr kann mit dem erfindungsgemäßen endoskopischen Chirurgiegerät bei Entweichen der Flüssigkeit aus der Submukosa einfach vom Schneidemodus auf den Injektionsmodus umgeschaltet und die Flüssigkeit in die Submukosa nachgefüllt werden. Die Operationszeiten können also wesentlich verkürzt werden.

Erfindungsgemäß weist die Einrichtung zur Zufuhr der mindestens einen Flüssigkeit eine, insbesondere flexible, Sonde mit mindestens zwei Arbeitskanälen auf, wobei in einem der Arbeitskanäle eine Düse und in einem anderen der Arbeitskanäle eine Hochfrequenz-Elektrode im Bereich des distalen Endes der Sonde angeordnet ist. Die Anordnung der Düse und der Hochfrequenz-Elektrode in zwei Arbeitskanälen einer Sonde bietet eine einfache Möglichkeit, die Einrichtung zu Zufuhr der mindestens einen Flüssigkeit und die Einrichtung zur Hochfrequenz-Chirurgie zur einheitlichen Handhabung zu kombinieren.

Die Einrichtung zur Hochfrequenz-Chirurgie kann eine Nadel, ein Haken, eine Nadel mit isolierter Spitze, eine Scheibe, eine Schlinge oder eine Schlinge mit isolierter Spitze umfassen, wodurch der Anwendungsbereich des Gerätes erweitert wird.

Die Ausbildung der HF-Einrichtung als Schlinge mit oder ohne isolierter Spitze ist besonders für die Polypektomie geeignet. Dabei bestand bisher das Problem, dass der Polyp von der Mukosa unter Umständen nicht ausreichend beanstandet war und nicht abgehoben werden konnte. Das heißt, dass die Schnitthöhe Ermessenssache der Chirurgen war und ausreichend viel vom Polypen abtragen musste, um kein Risikomaterial im Körper zu belassen, das zu späterer Zeit entarten konnte. Der Chirurg musste aber auch einen ausreichend großen Abstand von der Submukosa einhalten, um das Gewebe der Darmwand nicht zu gefährden (Perforationsgefahr).

Die Kombination einer HF-Schlinge mit einer Einrichtung zur Zufuhr einer Flüssigkeit ermöglicht die Injektion eines Fluidstahles unter den Polypen, so dass dieser ausreichend von der Submukosa beabstandet wird. Der Polyp kann dann sicher mit der Schlinge ektomiert bzw. resektiert werden, ohne dass das Gewebe der Darmwand beschädigt wird. Dabei können alle Arten von Schlingen, d.h. symmetrische oder asymmetrische Schlingen mit oder ohne isolierten Spitzen mit unterschiedlichen Formen oder Durchmessern eingesetzt werden.

Das Chirurgiegerät weist einen Handgriff auf mit einem Schalter, insbesondere einem Schiebeschalter, zum Freischalten der Einrichtung zur Hochfrequenz-Chirurgie und einem weiteren gesonderten Schalter, insbesondere einem Fußschalter, zur Aktivierung der Einrichtung zur Hochfrequenz-Chirurgie. Dadurch müssen zwei räumlich getrennte Schalter betätigt werden, wodurch die Einrichtung zur Hochfrequenz-Chirurgie freigeschaltet wird und daraufhin durch Betätigung des weiteren Schalters aktiviert wird. Auf diese Weise ist sichergestellt, dass die Einrichtung zur Hochfrequenz-Chirurgie nicht versehentlich betätigt wird.

Der weitere Schalter kann sowohl zur Aktivierung der Einrichtung zur Hochfrequenz-Chirurgie, als auch zur Aktivierung der Einrichtung zur Zufuhr der mindestens einen Flüssigkeit angepasst sein, so dass durch den weiteren Schalter, insbesondere den Fußschalter, sowohl die Zufuhr der Flüssigkeit als auch die Hochfrequenz-Elektrode betätigt werden können.

Die Sonde kann einen Kunststoffschlauch umfassen, der eine besonders einfache und preiswerte Möglichkeit gibt, die Sonde flexibel zu gestalten. Alternativ kann die Sonde ein flexibles Metallrohr umfassen, das auf dem Außenumfang isoliert ist. Durch den Metallinnenkern kann der Sondendurchmesser deutlich reduziert werden, ohne dass die Flexibilität dabei stark leidet.

Die Erfindung wird nachfolgend mit weiteren Einzelheiten unter Bezug auf die beigefügten schematischen Zeichnungen beispielsweise näher beschrieben.

In diesen zeigen
- Fig. 1a den Längsschnitt des distalen Endes einer Sonde eines erfindungsgemäßen Ausführungsbeispiels mit einer Hochfrequenz-Nadel;
- Fig. 1b einen Querschnittdurch die Sonde nach Fig. 1a;
- Fig. 2a den Längsschnitt durch das distale Ende einer Sonde eines weiteren erfin- dungsgemäßen Ausführungsbeispiels mit einem Hochfrequenz-Haken;
- Fig. 2b einen Querschnittdurch die Sonde nach Fig. 2a;
- Fig. 3 den Längsschnitt durch das distale Ende einer Sonde nach einem weiteren erfindungsgemäßen Ausführungsbeispiel mit einer Hochfrequenz-Nadel mit isolierter Spitze und
- Fig. 4 eine Seitenansicht eines Handgriffes mit einem Fluidanschluss und einem Hochfrequenzanschluss und
- Fig. 5 den Ablauf eines endoskopischen Eingriffes mit einem erfindungsgemäßen Chirurgiegerät.
- Fig. 6 eine perspektivische Ansicht eines endoskopischen Chirurgiegeräts, das im Injektionsmodus betrieben wird;
- Fig. 7 eine perspektivische Ansicht eines endoskopischen Chirurgiegeräts, das im HF-Modus betrieben wird;
- Fig. 8 eine perspektivische Ansicht eines endoskopischen Chirurgiegeräts, bei dem die Sonde eine HF-Schlinge mit isolierter Spitze aufweist, und
- Fig. 9 einen Längsschnittdurch eine Sonde gemäß einem erfindungsgemäßen Ausführungsbeispiel mit einer HF-Schlinge ohne isolierter Spitze.

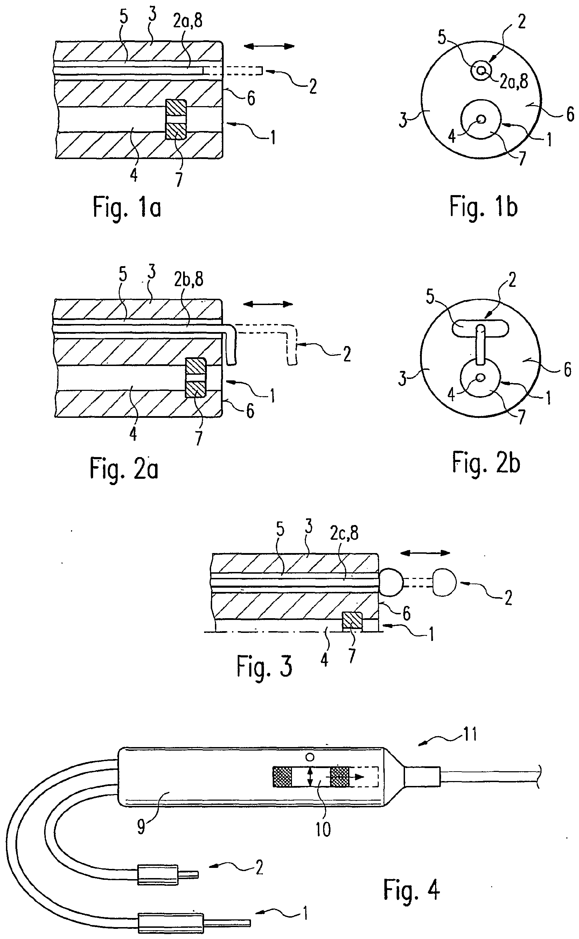

In Fig. 1a ist das distale Ende 6 einer Sonde 3 eines Ausführungsbeispiels des erfindungsgemäßen endoskopischen Chirurgiegerätes, bzw. Endoskops 11 dargestellt. Im Längsschnitt nach Fig. 1 ist dargestellt, dass die Sonde 3 zwei Arbeitskanäle 4, 5 umfasst. In dem einen der beiden Arbeitskanäle 4 ist eine Düse 7 fest angeordnet. Durch diesen Arbeitskanal 4 kann mindestens eine Flüssigkeit, insbesondere eine NaCl-Lösung zugeführt werden. Der Arbeitskanal 4 ist somit Teil der Einrichtung 1 zur Zufuhr mindestens einer Flüssigkeit. Diese Einrichtung 1 umfasst ferner einen Vorratsbehälter für die Flüssigkeit sowie eine Pumpenanordnung, durch die die Flüssigkeit unter Druck durch den Arbeitskanal 4 gepresst wird.

Ferner kann die Einrichtung 1 zur Zufuhr der mindestens einen Flüssigkeit, d.h. die Einrichtung zur Wasserstrahlchirurgie, durch eine entsprechende Drucksteuerung vom Injektionsmodus in den Dissektionsmodus und umgekehrt umgeschaltet werden. Im Injektionsmodus fungiert die Einrichtung 1 als nadelloser Injektor nach den aus der DE 2 333 720 A1 und der US 5,116,313 bekannten Prinzipien.

In der Sonde 3 ist außerdem eine Einrichtung 2 zur Hochfrequenz-Chirurgie vorgesehen. Diese Einrichtung 2 zur Hochfrequenz-Chirurgie umfasst eine Hochfrequenz-Elektrode 8, die in einem weiteren Arbeitskanal 5 in der Sonde 3 vorgesehen ist. Die beiden Arbeitskanäle 4, 5 sind im Wesentlichen parallel zueinander angeordnet.

Die Erfindung ist nicht auf zwei Arbeitskanäle beschränkt, sondern kann noch weitere Arbeitskanäle, insbesondere 3 oder 4 oder mehr Arbeitskanäle umfassen, wobei den verschiedenen Arbeitskanälen unterschiedliche Funktionen zugeordnet sein können: Beispielsweise könnten für den Injektionsmodus und den Dissektionsmodus zwei gesonderte Arbeitskanäle vorgesehen sein, zusätzlich zu dem für die Hochfrequenz-Elektrode 8 vorgesehenen Arbeitskanal. Auf diese Weise könnte ein mit einer Nadel versehener Injektor, die Wasserstrahldüse 7 und die HF-Elektrode 8 über 3 separate Arbeitskanäle angesteuert werden.

Außerdem sind im Endoskop 11 Einrichtungen zur optischen Überwachung des Eingriffs, wie beispielsweise Lichtleiter, vorgesehen (nicht gezeigt).

Je nach Anwendungsgebiet kann die Hochfrequenz-Elektrode 8 eine Hochfrequenz-Nadel 2a (Fig. 1a), einen drehfest angeordneten Hochfrequenz-Haken 2b bzw. -Spatel (Fig. 2ä), eine Hochfrequenz-Nadel mit isolierter Spitze 2c, insbesondere einer Keramikspitze (Fig. 3) oder eine nicht dargestellte Scheibe umfassen.

Das Endoskop 11 umfasst ferner einen Handgriff 9, der in Fig. 4 dargestellt ist. Der Handgriff 9 weist einen Anschluss der Einrichtung 2 zur Hochfrequenz-Chirurgie auf, der mit einer Hochfrequenz-Einheit (nicht dargestellt) verbunden werden kann. Der Handgriff 9 weist ferner mindestens einen Fluidanschluss der Einrichtung 1 zur Zufuhr der mindestens einen Flüssigkeit auf, der mit einer ebenfalls nicht dargestellten Wasserstrahlchirurgieeinheit verbunden werden kann. Die beiden Anschlussleitungen sind durch den Handgriff 9 geführt und münden in das Endoskop 11.

Der Handgriff 9 weist ferner einen Schalter 10, insbesondere einen Schiebeschalter auf, durch dessen Betätigung die Hochfrequenz-Elektrode 8 positioniert und freigeschaltet werden kann. Zur Positionierung wird die Hochfrequenz-Elektrode 8 aus der Sonde 3 herausgefahren, wie durch den Doppelpfeil und die gestrichelte Darstellung der Elektrode 8 in den Figuren 1a, 2a und 3 dargestellt ist. Die Aktivierung der Hochfrequenz-Elektrode 8 erfolgt über einen nicht dargestellten Fußschalter, wodurch sicher verhindert wird, dass die Elektrode 8 ungewollt betätigt wird. Die Aktivierung des Wasserstrahls kann über den selben Fußschalter erfolgen.

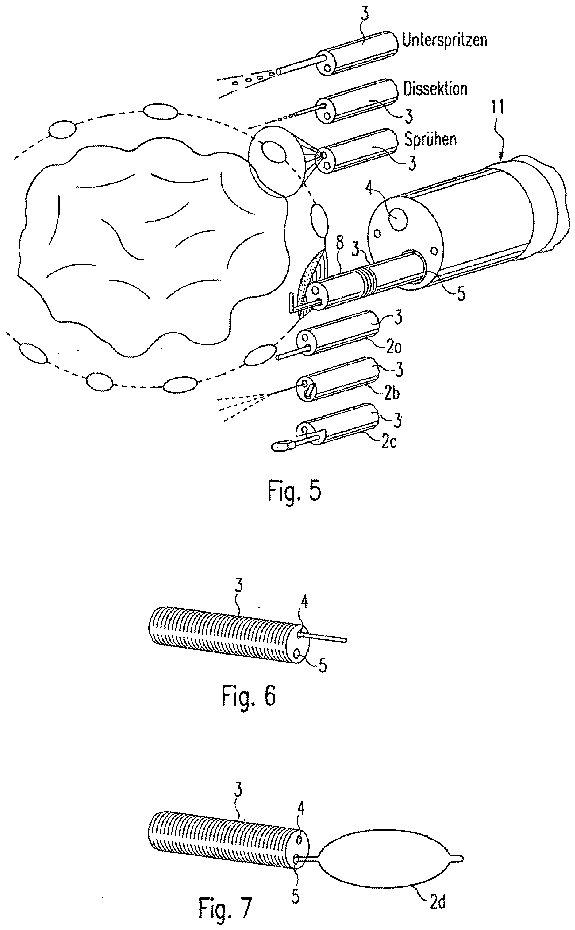

Das erfindungsgemäße Chirurgiegerät, bzw. Endoskop 11, wird wie folgt verwendet. In Fig. 5 ist dargestellt, dass zunächst die Wasserstrahlchirurgiefunktion des Chirurgiegerätes aktiviert wird, wobei durch den Arbeitkanal 4 und die Düse 7 der Sonde 3 ein feiner Injektionsstrahl erzeugt wird, so dass Flüssigkeit, insbesondere eine NaCl-Lösung unter die Mukosa in die Submukosa eingespritzt wird. Dadurch wird unter der Mukosa ein Flüssigkeitskissen gebildet, so dass die Mukosa von der Muscularis abgehoben wird. Im nächsten Schritt wird vom Injektionsmodus auf den Dissektionsmodus des Chirurgiegerätes umgestellt, wobei der Druck des Wasserstrahles erhöht wird, so dass die Mukosa resektiert werden kann. Wenn erforderlich, kann durch den Arbeitskanal 4, d.h. im Rahmen der Wasserstrahlchirurgiefunktion des erfindungsgemäßen Endoskops 11 Flüssigkeit auf den Eingriffsbereich gesprüht werden.

Daraufhin wird das Gerät von der Wasserstrahlfunktion auf die Hochfrequenz-Chirurgiefunktion umgeschaltet und die Elektrode 8 positioniert. Durch Aktivierung der Hochfrequenz-Elektrode 8 wird die Submukosa abgetrennt, wobei auch die koagulierende Wirkung der Elektrode 8 zum Tragen kommt.

Auf diese Weise ist es möglich, mit Hilfe eines einzigen Gerätes die Mukosa ausreichend weit von der Muscularis abzuheben und zu ektomieren, ohne dass die Magen-oder Darmwand beschädigt wird. Insbesondere ist es mit dem erfindungsgemäßen Chirurgiegerät ohne Gerätewechsel möglich, Flüssigkeit in die Submukosa nachzuspritzen, wenn die Submukosa im Lauf der Operation zu viel Flüssigkeit verliert, um die Mukosa ausreichend weit von der Magen- und Darmwand entfernt zu halten.

Die Sonde 3 ist flexibel, beispielsweise als Kunststoffschlauch oder als flexibles Metallrohr, das außen durch einen Kunststoffschlauch isoliert ist, ausgeführt.

Das Gerät gemäß den Figuren 6 bis 9 ist besonders für die Polypektomie geeignet und umfasst eine Sonde 3 mit zwei Arbeitskanälen 4, 5, von denen der eine Arbeitskanal 4 zur Injektion eines Fluidstrahles und der andere Arbeitskanal 5 zur Aufnahme einer HF-Schlinge dient. Die beiden Arbeitskanäle sind in eine Sonde 3 zur einheitlichen Handhabung integriert. Die einheitliche Handhabung der Injektions- und Schneideinrichtung kann bei dem Gerät nach den Figuren 6 bis 9 ebenso wie bei den erfindungsgemäßen Ausführungsbeispielen auch dadurch erreicht werden, dass zwei separate Schläuche mit jeweils einem Arbeitskanal 4, 5 zu einer Sonde verbunden werden.

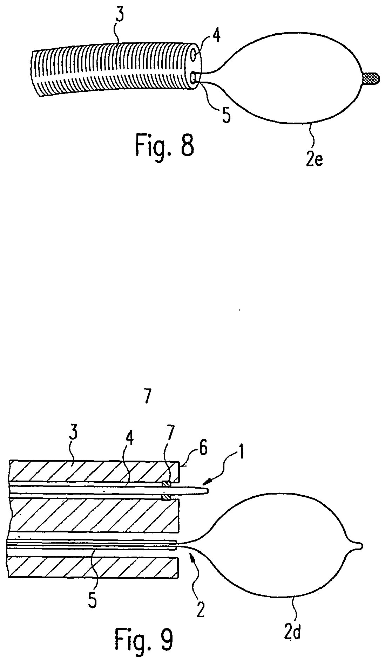

Der Aufbau der Sonde gemäß Fig. 9 entspricht im Wesentlichen dem bereits erläuterten Aufbau der Sonde gemäß den vorstehend beschriebenen Ausführungsbeispielen, wobei in einem der beiden Arbeitskanäle 4 eine Düse 7 angeordnet ist, durch die der Fluidstrahl zur Injektion bzw. Dissektion austritt. Im anderen der beiden Arbeitskanäle 5 ist die HF-Schlinge angeordnet, die in den Arbeitskanal 5 eingezogen bzw. aus dem Arbeitskanal 5 ausgeschoben werden kann.

Als Schlingen eignen sich alle Arten von Schlinge mit unterschiedlichen Formen und Durchmessern sowie Schlingen 2e mit isolierter Spitze und Schlingen 2d ohne isolierter Spitze. Ferner können symmetrische oder asymmetrische und/oder drehbare oder drehfeste Schlingen verwendet werden.

Die Funktion der Sonden 3 gemäß den Ausführungsbeispielen nach den Figuren 6 bis 9 entspricht im Wesentlichen der Funktion der vorstehend beschriebenen Ausführungsbeispielen, wobei im Injektionsmodus ein Fluidstrahl, vorzugsweise 0,9 %ige NaCl-Lösung in die Submukosa unter den Polypen injiziert wird, so dass dieser ausreichend von der Submukosa beabstandet wird. Daraufhin wird der Polyp mittels der Schlinge 2d, 2e resektiert bzw. ektomiert.

### Bezugszeichen

- 1: Einrichtung zur Zufuhr mindestens einer Flüssigkeit
- 2: Einrichtung zur Hochfrequenz-Chirurgie
- 2a: Nadel
- 2b: Haken
- 2c: Nadel mit isolierter Spitze
- 2d: Schlinge ohne isolierte Spitze
- 2e: Schlinge mit isolierter Spitze
- 3: Sonde
- 4, 5: Arbeitskanäle
- 6: distales Ende der Sonde
- 7: Düse
- 8: Hochfrequenz-Elektrode
- 9: Handgriff
- 10: Schalter
- 11: Endoskop

## Patentansprüche

1. Endoskopisches Chirurgiegerät für die endoskopische Mukosaresektion mit einer Einrichtung (1) zur Zufuhr mindestens einer Flüssigkeit und einer Einrichtung (2) zur Hochfrequenz-Chirurgie, die mit der Einrichtung (1) zur Zufuhr der mindestens einen Flüssigkeit zur einheitlichen Handhabung kombiniert ist, wobei die Einrichtung (1) zur Zufuhr der mindestens einen Flüssigkeit für eine nadellose Injektion und/oder Dissektion ausgebildet ist,
**dadurch gekennzeichnet, dass**
die Einrichtung (1) zur Zufuhr der mindestens einen Flüssigkeit eine Sonde (3) mit mindestens zwei Arbeitskanälen (4,5) aufweist, wobei in einem der Arbeitskanäle (4) eine Düse (7) und in einem anderen der Arbeitskanäle (5) eine Hochfrequenz-Elektrode (8) im Bereich des distalen Endes (6) der Sonde (3) herausfahrbar angeordnet ist, wobei ein Handgriff (9) mit einem Schalter (10) zum Freischalten der Einrichtung (2) zur Hochfrequenz-Chirurgie und ein weiterer gesonderter Schalter zur Aktivierung der Einrichtung (2) zur Hochfrequenz-Chirurgie vorgesehen sind.

2. Chirurgiegerät nach Anspruch 1,
**dadurch gekennzeichnet, dass**
der weitere Schalter sowohl zum Aktivieren der Einrichtung (2) zur Hochfrequenz-Chirurgie als auch zur Aktivierung der Einrichtung (1) zur Zufuhr der mindestens einen Flüssigkeit angepasst ist.

3. Chirurgiegerät nach Anspruch 1 oder 2,
**dadurch gekennzeichnet, dass**
der Handgriff (9) einen Schiebeschalter umfasst.

4. Chirurgiegerät nach einem der Ansprüche 1 bis 3,
**dadurch gekennzeichnet, dass**
der gesonderte Schalter ein Fußschalter ist.

5. Chirurgiegerät nach einem der Ansprüche 1 bis 4,
**dadurch gekennzeichnet, dass** die Sonde (3) einen Kunststoffschlauch umfasst.

6. Chirurgiegerät nach einem der Ansprüche 1 bis 4,
**dadurch gekennzeichnet, dass**
die Sonde (3) ein flexibles Metallrohr umfasst, das auf dem Außenumfang isoliert ist.

## Claims

1. Endoscopic surgical instrument for endoscopic mucosal resection, having a device (1) for supplying at least one fluid and a device (2) for high-frequency surgery which is combined with the device (1) for supplying the at least one fluid for uniform handling, the device (1) for supplying the at least one fluid being arranged for needleless injection and/or dissection,
**characterized in that**
the device (1) for supplying the at least one fluid has a probe (3) having at least two working channels (4, 5), a nozzle (7) being arranged in one of the working channels (4) and a high-frequency electrode (8) being arranged in another of the working channels (5) so as to be movable outwards in the region of the distal end (6) of the probe (3), a handle (9) being provided having a switch (10) for enabling the device (2) for high-frequency surgery and a further separate switch for activating the device (2) for high-frequency surgery.

2. Surgical instrument according to claim 1,
**characterized in that**
the further switch is adapted both for activation of the device (2) for high-frequency surgery and for activation of the device (1) for supplying the at least one fluid.

3. Surgical instrument according to claims 1 or 2,
**characterized in that**
the handle (9) comprises a slide switch.

4. Surgical instrument according to any one of claims 1 to 3,
**characterized in that**
the separate switch is a foot switch.

5. Surgical instrument according to any one of claims 1 to 4,
**characterized in that**
the probe (3) comprises plastics tubing.

6. Surgical instrument according to any one of claims 1 to 4,
**characterized in that**
the probe (3) comprises a flexible metal tube which is insulated on its outer periphery.

## Revendications

1. Appareil chirurgical endoscopique pour la résection endoscopique des muqueuses, comprenant un dispositif (1) pour l'alimentation d'au moins un liquide et un dispositif (2) pour la chirurgie à haute fréquence qui est combiné avec le dispositif (1) pour l'alimentation du au moins un liquide pour une manipulation uniforme, le dispositif (1) pour l'alimentation du au moins un liquide étant conçu pour une injection et/ou dissection sans aiguille, **caractérisé en ce que**
le dispositif (1) d'alimentation du au moins un liquide comporte une sonde (3) avec au moins deux canaux de travail (4, 5), une buse (7) étant disposée dans un des canaux de travail (4) et une électrode haute fréquence (8) étant disposée dans un autre des canaux de travail (5) dans la zone de l'extrémité distale (6) de la sonde (3), de manière à pouvoir sortir, avec une poignée (9) munie d'un interrupteur (10) pour libérer le dispositif (2) pour la chirurgie à haute fréquence et un autre interrupteur distinct pour activer le dispositif (2) pour la chirurgie à haute fréquence sont prévus.

2. Appareil chirurgical selon la revendication 1,
**caractérisé en ce que**
l'interrupteur supplémentaire est adapté à la fois pour activer le dispositif (2) pour la chirurgie à haute fréquence et pour activer le dispositif (1) pour alimenter le au moins un liquide.

3. Appareil chirurgical selon la revendication 1 ou 2,
**caractérisé en ce que**
la poignée (9) comprend un interrupteur coulissant.

4. Appareil chirurgical selon l'une des revendications 1 à 3,
**caractérisé en ce que**
l'interrupteur séparé est un interrupteur au pied.

5. Appareil chirurgical selon l'une des revendications 1 à 4,
**caractérisé en ce que**
la sonde (3) comprend un tube en plastique.

6. Appareil chirurgical selon l'une des revendications 1 à 4,
**caractérisé en ce que**
la sonde (3) comprend un tube métallique flexible isolé sur la périphérie extérieure.
